(19) **Europäisches Patentamt / European Patent Office / Office européen des brevets**

(11) **EP 1 746 959 B1**

(12) **EUROPEAN PATENT SPECIFICATION**

(45) Date of publication and mention
of the grant of the patent:
**24.11.2010 Bulletin 2010/47**

(21) Application number: **05722548.4**

(22) Date of filing: **26.01.2005**

(51) Int Cl.:
***A61F 13/15*** (2006.01)

(86) International application number:
**PCT/US2005/002438**

(87) International publication number:
**WO 2005/110311 (24.11.2005 Gazette 2005/47)**

(54) **ABSORBENT GARMENTS WITH FORM-FITTING PROPERTIES**

SAUGFÄHIGE KLEIDUNGSSTÜCKE MIT FORMANPASSUNGSEIGENSCHAFTEN

VETEMENTS ABSORBANTS AYANT DES PROPRIETES D'AJUSTEMENT

(84) Designated Contracting States:
**DE FR GB NL**

(30) Priority: **29.04.2004 US 835528**

(43) Date of publication of application:
**31.01.2007 Bulletin 2007/05**

(73) Proprietor: **KIMBERLY-CLARK WORLDWIDE, INC.**
**Neenah, WI 54956 (US)**

(72) Inventors:
• **SAWYER, Lawrence, Howell**
**Neenah, WI 54956 (US)**
• **POPP, Robert, Lee**
**Hortonville, WI 54944 (US)**
• **OLSON, Christopher, Peter**
**Neenah, WI 54956 (US)**

• **CARR, James, Marcus**
**Kaukauna, WI 54130 (US)**
• **MLEZIVA, Mark, Michael**
**Appleton, WI 54913 (US)**
• **FAULKS, Michael, John**
**Neenah, WI 54956 (US)**

(74) Representative: **Mabey, Katherine Frances**
**Dehns**
**St Bride's House**
**10 Salisbury Square**
**London**
**EC4Y 8JD (GB)**

(56) References cited:
**WO-A-02/34182      WO-A-97/22318**
**WO-A-03/057106     US-A- 5 690 627**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

## Background of the Invention

Absorbent articles such as diapers, training pants, incontinence garments, swim undergarments, and the like conventionally include a liquid permeable body-facing liner, a liquid impermeable outer cover, and an absorbent core. The absorbent core is typically located in between the outer cover and the liner for taking in and retaining liquids (e.g., urine) exuded by the wearer.

In some of these absorbent articles, the articles contain various elastic materials to permit some expansion of the article when necessary to provide a better fit on the wearer. The elastic members are also designed to contract when being worn in order to provide the article with form-fitting properties at least in some areas. During use, the article is subjected to forces such as those generated by loading of the absorbent article and movement of the wearer. In some Instances, after the absorbent article has been insulted with a liquid, the crotch area of the article may begin to droop or sag.

WO97/22318 discloses an absorbent article containing extensible zones. WO03/057106 discloses an absorbent article. US 5,890,627 discloses an absorbent article with fit enhancement system.

In this regard, improvements are needed In constructing absorbent articles that have form-fitting properties, even after the article has absorbed substantial amounts of liquid. In particular, a need exists for an absorbent article that does not droop or sag in the crotch area after being wetted. A need also exists for an absorbent article that has improved donning characteristics.

The present invention provides an absorbent article in accordance with claim 1.

In general, the present invention relates to disposable absorbent articles having carefully controlled stretch properties. For Instance, the absorbent articles may have form-fitting properties resulting in an improved fit and appearance. The carefully controlled stretch properties of the articles prevent against sagging or drooping in the crotch region, even after the article has been wetted. Specifically, the crotch region is maintained In close contact with the body during use.

The present invention is directed to an absorbent article comprising an outer cover. The outer cover is stretchable and elastic and comprises an elastic material. A stretchable bodyside liner is joined to the outer cover in a superimposed relation. The liner may be elastic in some applications.

An absorbent structure is positioned in between the outer cover and the liner. The outer cover, liner and absorbent structure form a chassis. The chassis defines a waist opening opposite two leg openings when worn about a wearer. In various embodiments, elastic members or other panels may be adhered to the chassis for forming the absorbent article.

In accordance with the present invention, the chassis comprises six equal distant zones in a longitudinal direction. The zones include a front waist zone, a back waist zone; a front midsection zone, a back midsection zone, a front crotch zone and a back crotch zone. The chassis is constructed such that the front crotch zone, the front midsection zone and the front waist zone have a lengthwise stretch of from about 5% to about 30%. The chassis is also constructed such that at least about 60%, such as at least about 80% of the above stretch Is contained in the front waist zone. By carefully controlling the stretch properties as described above, the present inventors have found that the article assumes better form-fitting properties and donning characteristics.

In one embodiment, the chassis Is constructed such that the back crotch zone, the back midsection zone, and the back waist zone also have a lengthwise stretch of from about 5% to about 30% and is constructed such that at least 80% of the stretch In the back zones is contained In the back waist zone. The stretch in the back zones may be more, the same or less than the stretch in the front zones. Further, in other embodiments, at least 85% of the stretch in the front zones and/or the back zones, and particularly at least 90% of the stretch in the front zones and/or the back zones is contained in the front and back waist zones, respectively.

The absorbent structure that is located between the outer cover and the liner is generally located in the front and back crotch zones and at least partially in the front and back midsection zones. In other embodiments, the absorbent structure may also extend into the front and/or back waist zones. The absorbent structure is used in order to control the stretch properties of the chassis. For instance, in one embodiment, the absorbent structure is attached to the liner, to the outer cover, or to both the liner and the outer cover within the front and back crotch zones and within the front and back midsection zones. Attaching the absorbent structure to the liner and/or the outer cover inhibits stretch of the liner and outer cover in the zones where the absorbent structure is attached, especially if the absorbent structure is less stretchable than the liner and/or the outer cover.

In embodiments, the absorbent structure is adhered to the liner and/or the outer cover using an adhesive, such as a hot melt adhesive.

In one particular embodiment of the present invention, the chassis may be constructed such that only the front waist zone and the back waist zone have stretch properties at lower tensions. At greater tensions in the longitudinal direction, however, the crotch zones and the midsection zones may be stretchable. For instance, In this embodiment, the crotch zones and the midsection zones may be stretchable only at tensions greater than about 100 g/cm. In this embodiment, for instance, the absorbent structure may be adhered to the chassis in a manner such that the attachment degrades at higher tensions to allow for stretching.

In one embodiment, the outer cover and the liner are both made from elastic materials. For instance, the outer cover may be made from an elastic laminate containing a nonwoven web. The liner, on the other hand, may comprise a liquid permeable elastic film or a nonwoven web containing an elastic material.

Other features and aspects of the present invention are discussed in greater detail below.

## Brief Description of the Drawings

A full and enabling disclosure of the present invention, including the best mode thereof to one skilled in the art, is set forth more particularly in the remainder of the specification, including reference to the accompanying figures, in which:

Figure 1 is a perspective view of one embodiment of an absorbent article made in accordance with the present invention;

Figure 2 is a plan view of the absorbent article shown in Figure 1 with the article in an unfastened, unfolded and laid flat condition showing the surface of the article that faces away from the wearer;

Figure 3 is a plan view similar to Figure 2 showing the surface of the absorbent article that faces the wearer when worn and with portions cut away to show underlying features;

Figure 4 is another embodiment of a plan view of an absorbent article made in accordance with the present invention showing the surface of the article that faces the wearer and with portions cut away to show underlying features;

Figure 5 is a plan view of the absorbent article shown in Figure 4 illustrating the stretch properties of the article;

Figure 6 is a plan view of another absorbent article made in accordance with the present invention showing the surface of the article that faces the wearer when worn and including cut away portions to show internal features;

Figure 7 is a plan view of the absorbent article shown in Figure 3 illustrating where one might obtain a sample from the chassis in order to test for stretch characteristics and tensile strength; and

Figure 8 is a plan view illustrating the sample obtained from the chassis shown in Fig. 7 severed into six equidistant sections for stretch testing.

Repeated use of reference characters in the present specification and drawings is intended to represent the same or analogous features or elements of the present invention.

## Detailed Description

It is to be understood by one of ordinary skill in the art that the present discussion is a description of exemplary embodiments only, and is not intended as limiting the broader aspects of the present invention.

In general, the present invention Is directed to absorbent articles designed to provide improved dry and wet fit while also providing improved ease of donning. The absorbent article may be, for instance, a diaper, a toilet training pant, an adult incontinence garment, a swim undergarment, or the like. Absorbent garments made according to the present invention have stretch in at least one dimension and may exhibit biaxial or multi-axial stretch properties. In particular, the absorbent articles have stretch properties at least in the longitudinal direction of the product or, when the product is being worn, from the crotch region of the product to the waist region.

More particularly, absorbent articles made in accordance with the present invention have a percentage of stretch in the longitudinal direction from the mid point of the crotch region to the front waistband and/or to the back waistband in the range of from about 5% to about 30%. Additionally, about 60%, such as about 80% of the stretch resides in a front waist zone and/or in a back waist zone. More particularly, at least about 85%, such as greater than about 90% of the stretch may reside In the front waist zone and/or the back waist zone. Thus, the crotch region and the lower front and back regions do not substantially contribute to the stretch properties of the product.

Through the above carefully controlled stretch properties, the present inventors have found that absorbent articles exhibit improved fit and appearance. In particular, the stretch properties provide form-fitting properties while also preventing sagging or drooping of the crotch region, even after the crotch region is wetted. In particular, the construction of the absorbent article maintains the article in close contact with the body, even after the article is insulted with a liquid. The construction of the article further allows for a customized fit to a user especially in the longitudinal direction. Specifically, the stretch properties of the article may accommodate broader or various user shapes. The construction has also been found to facilitate donning of the product.

In general, the absorbent articles are made with stretchable and/or elastic materials. As used herein, the term "stretchable" refers to a material that may be extensible and/or elastic (or.elastomeric). That is, the material may be extended, deformed or the like, without breaking, and may or may not significantly retract after removal of an extending force. The terms "elastic" or "elastomeric" are used interchangeably herein and refer to a property of a material where upon removal of an elongating force, the material was capable of recovering to substantially its unstretched size and shape or the material exhibits a significant retractive force. The term "extensible" refers to a property of a material where upon removal of an elongating force, the material experiences a substantially permanent deformation or the material does not exhibit a significant retractive force. In particular, elastic materials utilized in connection with the present invention may be elongated/extended or stretched In at least one direction without breaking by at least 15%, such as by at least 25% (to at least 125% of its initial unstretched length) in at least one direction, suitably by at least 50% (to at least 150% of its initial unstretched length) and which will recover, upon release of the applied stretching or biasing force, at least 10% of their elongation. It is generally advantageous that the elastomeric material or composite be capable of being elongated by at least 100%, more desirably at least 200%, of its relaxed length and recover at least 30% and more desirably 50% of its elongation upon release of a stretching, biasing force, within about one minute.

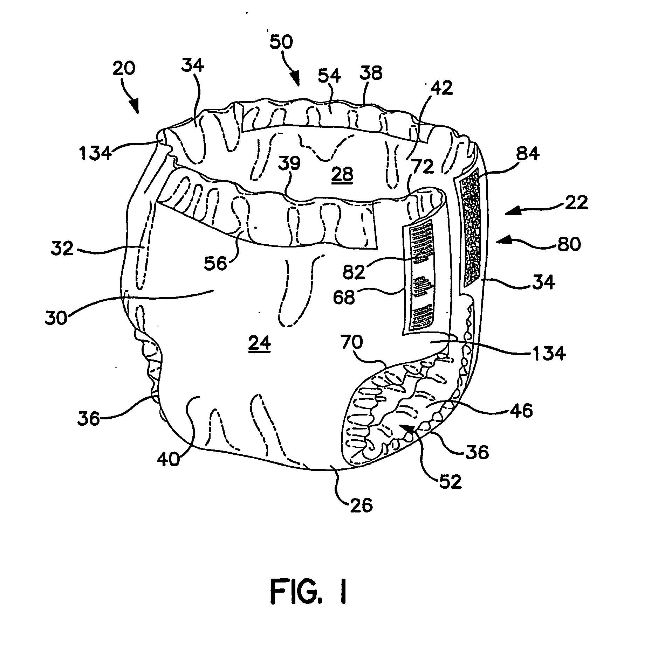

Referring to **Fig. 1,** for exemplary purposes, an absorbent article **20** that may be made in accordance with the present invention is shown. The absorbent article **20** may or may not be disposable, which refers to articles that are intended to be discarded after a limited period of use instead of being laundered or otherwise conditioned for reuse. It is understood that the present invention is suitable for use with various other absorbent articles intended for personal wear, including but not limited to diapers, feminine hygiene products, incontinence products, medical garments, surgical pads and bandages, other personal care or health care garments, and the like without departing from the scope of the present invention.

By way of illustration only, various materials and methods for constructing training pants such as the pants 20 of the various aspects of the present invention are disclosed in PCT Patent Application WO 00/37009 published June 29, 2000 by A. Fletcher et al; U.S. Patent 4,940,464 issued July 10, 1990 to Van Gompel et al.; U.S. Patent 5,766,389 issued June 16, 1998 to Brandon et al., and U.S. Patent 6,645,190 issued November 11, 2003 to Olson et al.

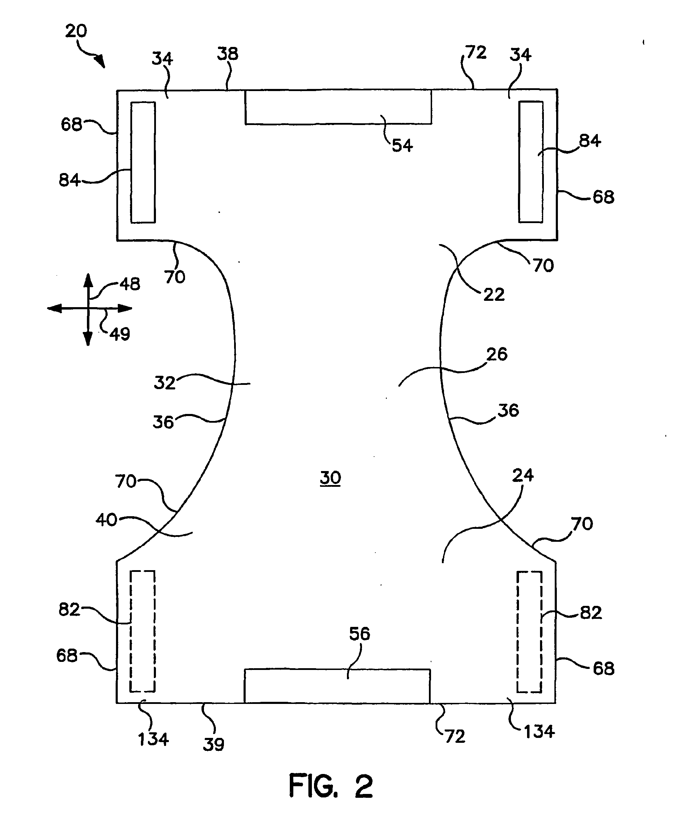

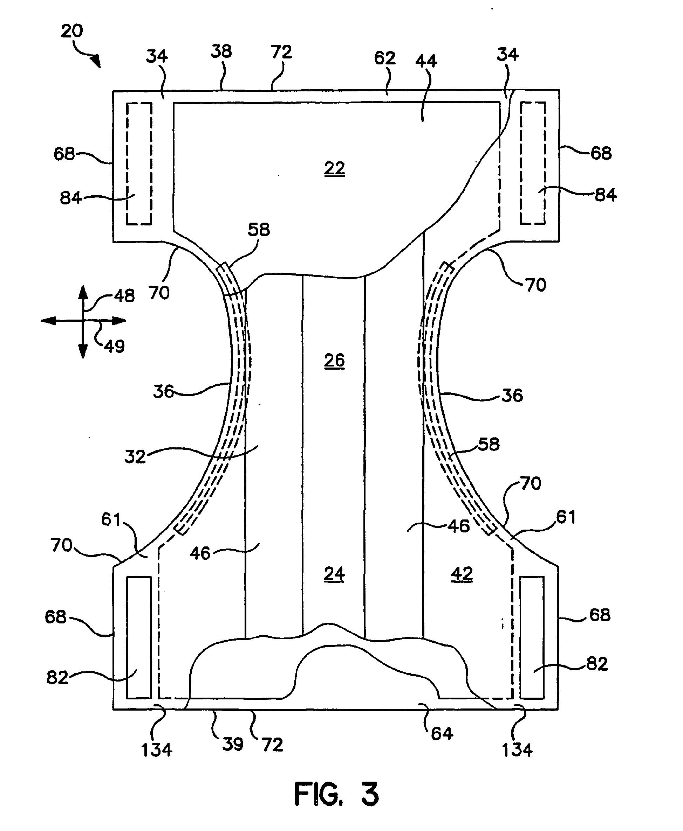

A pair of training pants **20** is representatively illustrated in **Fig. 1** in a partially fastened condition. The training pants **20** shown in **Fig. 1** is also represented in **Figs. 2** and **3** in an opened and unfolded state. Specifically, **Fig. 2** is a plan view illustrating the exterior side of the pants **20,** while **Fig. 3** illustrates the interior side of the pants **20.** As shown in **Figs. 2** and **3,** the pants **20** defines a longitudinal direction **48** that extends from the front of the training pants when worn to the back of the training pants. Opposite to the longitudinal direction **48** is a lateral direction **49.**

The pants **20** define a pair of longitudinal end regions, otherwise referred to herein as a front region **22** and a back region **24,** and a center region, otherwise referred to herein as a crotch region **26,** extending longitudinally between and interconnecting the front and back regions **22, 24.** The pant **20** also defines an inner surface **28** adapted in use (e.g., positioned relative to the other components of the pants **20**) to be disposed toward the wearer, and an outer surface **30** opposite the inner surface. The front and back regions **22, 24** are those portions of the pants **20,** which when worn, wholly or partially cover or encircle the waist or mid-lower torso of the wearer. The crotch region **26** generally is that portion of the pants **20** which, when worn, is positioned between the legs of the wearer and covers the lower torso and crotch of the wearer. The training pants **20** have a pair of laterally opposite side edges **36** and a pair of longitudinally opposite waist edges, respectively designated front waist edge **38** and back waist edge **39.**

The illustrated pants **20** may include a chassis **32,** a pair of laterally opposite front side panels **34** extending laterally outward at the front region **22** and a pair of laterally opposite back side panels **134** extending laterally outward at the back region **24.**

Referring to **Figs. 1-3,** the chassis **32** includes an outer cover **40** and a bodyside liner **42 (Figs. 1** and **3)** that may be joined to the outer cover **40** in a superimposed relation therewith by adhesives, ultrasonic bonds, thermal bonds or other conventional techniques. Referring to **Fig. 3,** the liner **42** may suitably be joined to the outer cover **40** along the perimeter of the chassis **32** to form a front waist seam **62** and a back waist seam **64.** As shown in **Fig. 3,** the liner **42** may suitably be joined to the outer cover **40** to form a pair of side seams **61** in the front region **22** and the back region **24.** The liner **42** can be generally adapted, i.e., positioned relative to the other components of the pants **20,** to be disposed toward the wearer's skin during wear of the pants. The chassis **32** may further include an absorbent structure **44** particularly shown in **Fig. 3** disposed between the outer cover **40** and the bodyside liner **42** for absorbing liquid body exudates exuded by the wearer, and may further include a pair of containment flaps **46** secured to the bodyside liner **42** for inhibiting the lateral flow of body exudates.

With the training pants **20** in the fastened position as partially illustrated in **Fig. 1,** the front and back side panels **34, 134** can be connected together by a fastening system **80** to define a three-dimensional pants configuration having a waist opening **50** and a pair of leg openings **52.** The front and back side panels **34** and **134,** upon wearing of the pants **20,** thus include the portions of the training pants **20** which are positioned on the hips of the wearer. The waist edges **38** and **39** of the training pants **20** are configured to encircle the waist of the wearer and together define a waist opening **50** of the pants.

The elasticized containment flaps **46** as shown in **Fig. 3** define a partially unattached edge which assumes an upright configuration in at least the crotch region **26** of the training pants **20** to form a seal against the wearer's body. The containment flaps **46** can extend longitudinally along the entire length of the chassis **32** or may extend only partially along the length of the chassis. Suitable constructions and arrangements for the containment flaps **46** are generally well known to those skilled in the art and are described in U.S. Patent 4,704,116 issued November 3, 1987 to Enloe.

To further enhance containment and/or absorption of body exudates, the training pants **20** may also suitably include a front waist elastic member **54 (Fig. 1),** a rear waist elastic member **56,** and leg elastic members **58 (Fig. 3),** as are known to those skilled in the art. The waist elastic members **54** and **56** can be operatively joined to the outer cover **40** and/or the bodyside liner **42** and can extend over part or all of the waist edges **38, 39.** The leg elastic members **58** can be operatively joined to the outer cover **40** and/or the bodyside liner **42** and positioned in the crotch region **26** of the training pants **20.**

The waist elastic members **54** and **56,** and the leg elastic members **58** can be formed of any suitable elastic material. As is well known to those skilled in the art, suitable elastic materials include sheets, strands or ribbons of natural rubber, synthetic rubber, or thermoplastic elastomeric polymers. The elastic materials can be stretched and adhered to a substrate, adhered to a gathered substrate, or adhered to a substrate and then elasticized or shrunk, for example with the application of heat, such that elastic retractive forces are imparted to the substrate. In one particular aspect, for example, the leg elastic members **58** may include a plurality of dry-spun coalesced multifilament spandex elastomeric threads sold under the trade name LYCRA and available from Invista, Wilmington, Delaware, U.S.A.

As shown in **Figs. 1 through 3,** the side panels **34** and **134** can be formed as an integral portion of the chassis **32.** For example, the side panels **34, 134** can include a generally wider portion of the outer cover **40,** the bodyside liner **42,** and/or other components of the chassis **32.** As described above, the side panels **34** and **134** may be attached together using any suitable fastening system **80.**

In the embodiments shown in the figures, the side panels **34** and **134** are releaseably attachable. It should be understood, however, that in other embodiments the side panels **34** and **134** may be permanently joined together. For instance, the side panels may be made from a unitary piece of material. Alternatively, the side panels may be bonded together using ultrasonic bonding, thermal bonding or an adhesive. In this embodiment, the absorbent article is pulled over the legs when being worn.

In an alternative embodiment of the present invention, the side panels **34** and **134** may be separately attached to the chassis **32.** For instance, the front side panels **34** can be permanently bonded to and extend transversely outward beyond the side margins of the chassis **32.** Similarly, the back side panels **134** can be permanently bonded to and extend transversely outward beyond the side margins of the chassis **32** and the back region **24.** The side panels **34** and **134** may be bonded to the chassis **32** using attachment means known to those skilled in the art such as adhesive, thermal or ultrasonic bonding.

The front and back side panels **34** and **134** each have a longitudinal outer edge **68,** and a leg end edge **70** disposed toward the longitudinal center of the training pants **20,** and waist end edges **72** disposed toward a longitudinal end of the training pants. The leg end edges **70** and the outer edges **68** of the side panels **34** and **134** form part of the pant side edges **36** of the training pants **20.** The leg end edges **70** of the absorbent article **20** may be suitably curved and/or angled relative to the lateral direction **49** to provide a better fit around the wearer's legs. However, it is understood that only one of the leg end edges **70** may be curved or angled, such as the leg end edge of the back region **24,** or alternatively, neither of the leg end edges may be curved or angled, without deporting from the scope of the present invention. The waist end edges **72** are suitably parallel to the transverse axis **49.** The waist end edges **72** of the front side panels **34** form part of the front waist edge **38** of the training pants **20,** and the waist end edges **72** of the back side panels **134** form part of the back waist edge **39** of the pants.

In configurations where the side panels **34, 134** are separately attached, the side panels may be provided by an elastic material capable of stretching at least In a direction generally parallel to the lateral direction **49** of the training pants **20.** Suitable elastic materials, as well as one process of incorporating elastic side panels into training pants, are described in the following U.S. Patents: 4,940,464 issued July 10, 1990 to Van Gompel et al.; 5,224,405 issued July 6, 1993 to Pohjola; 5,104,116 issued April 14, 1992 to Pohjola; and 5,046,272 Issued September 10, 1991 to Vogt et al. In particular aspects, the elastic material may include a stretch-thermal laminate (STL), a neck-bonded laminate (NBL), a reversibly necked laminate, or a stretch-bonded laminate (SBL) material. Methods of making such materials are well known to those skilled in the art and described In U.S. Patent 4,663,220 issued May 5, 1987 to Wisneski et al.; U.S. Patent 5,226,992 issued July 13, 1993 to Morman; European Patent Application No. EP 0 217 032 published on April 8, 1987 in the name of Taylor et al.; and PCT application WO 01/88245 in the name of Weich et al. Alternatively, the side panel material may include other woven or non-woven materials, such as those described later herein as being suitable for construction of the outer cover **40** and/or the bodyside liner **42;** mechanically pre-strained composites; or stretchable but inelastic materials.

The fastening system **80** may include laterally opposite first fastening components **82** adapted for refastenable engagement to corresponding second fastening components **84.** In one aspect, a front or outer surface of each of the fastening components **82, 84** includes a plurality of engaging elements. The engaging elements of the first fastening components **82** are adapted to repeatedly engage and disengage corresponding engaging elements of the second fastening components **84** to releasably secure the pants **20** In its three-dimensional configuration.

The fastening components **82, 84** may be any refastenable fasteners suitable for absorbent articles, such as adhesive fasteners, cohesive fasteners, mechanical fasteners, or the like. In particular aspects the fastening components include mechanical fastening elements for improved performance. Suitable mechanical fastening elements can be provided by interlocking geometric shaped materials, such as hooks, loops, bulbs, mushrooms, arrowheads, balls on stems, male and female mating components, buckles, snaps, or the like.

In the illustrated aspect, the first fastening components 82 include loop fasteners and the second fastening components 84 Include complementary hook fasteners. Alternatively, the first fastening components 82 may include hook fasteners and the second fastening components 84 may be complementary loop fasteners. In another aspect, the fastening components 82, 84 can be interlocking similar surface fasteners, or adhesive and cohesive fastening elements such as an adhesive fastener and an adhesive-receptive landing zone or material; or the like. Although the training pants 20 illustrated in Fig. 1 indicate the back side panels 134 overlapping the front side panels 34 upon connection thereto, which is convenient, the training pants 20 can also be configured so that the front side panels 34 overlap the back side panels 134 when connected. One skilled in the art will recognize that the shape, density and polymer composition of the hooks and loops may be selected to obtain the desired level of engagement between the fastening components 82, 84. Optionally, either one or both of the fastening components 82, 84 may be provided by one of the inner or outer surfaces 28 and 30 of the side panels 34 and 134. Suitable fastening systems are also disclosed in the previously incorporated PCT Patent Application WO 00/37009 published June 29, 2000 by A. Fletcher et al.

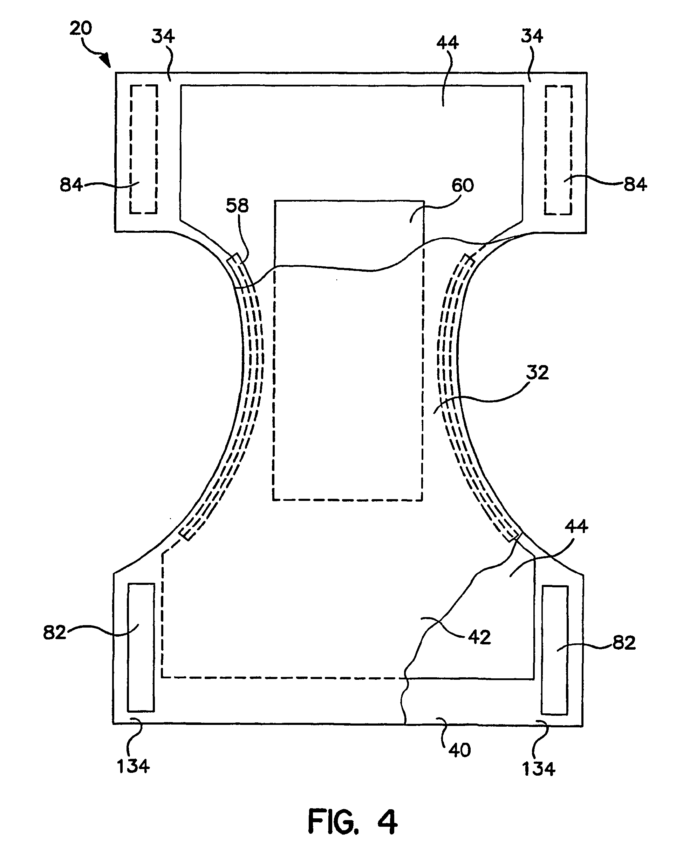

Referring to Fig. 4, another embodiment of an absorbent article 20 made in accordance with the present invention is illustrated. In Fig. 4, the absorbent article 20 is shown in an unfolded state illustrating the interior surface of the article, which faces the wearer during use. In Fig. 4, the absorbent article 20 further includes a surge management layer 60 which may be optionally located adjacent the absorbent structure 44 and attached to various components in the article 20 such as the absorbent structure 44 or the bodyside liner 42 by methods known in the art, such as by using an adhesive. A surge management layer 60 helps to decelerate and diffuse surges or gushes of liquid that may be rapidly introduced into the absorbent structure of the article. Desirably, the surge management layer can rapidly accept and temporarily hold the liquid prior to releasing the liquid Into the storage or retention portions of the absorbent structure. Examples of suitable surge management layers are described in U.S. Pat. No. 5,486,166; and U.S. Pat. No. 5,490,846. Other suitable surge management materials are described in U.S. Pat. No. 5,820,973.

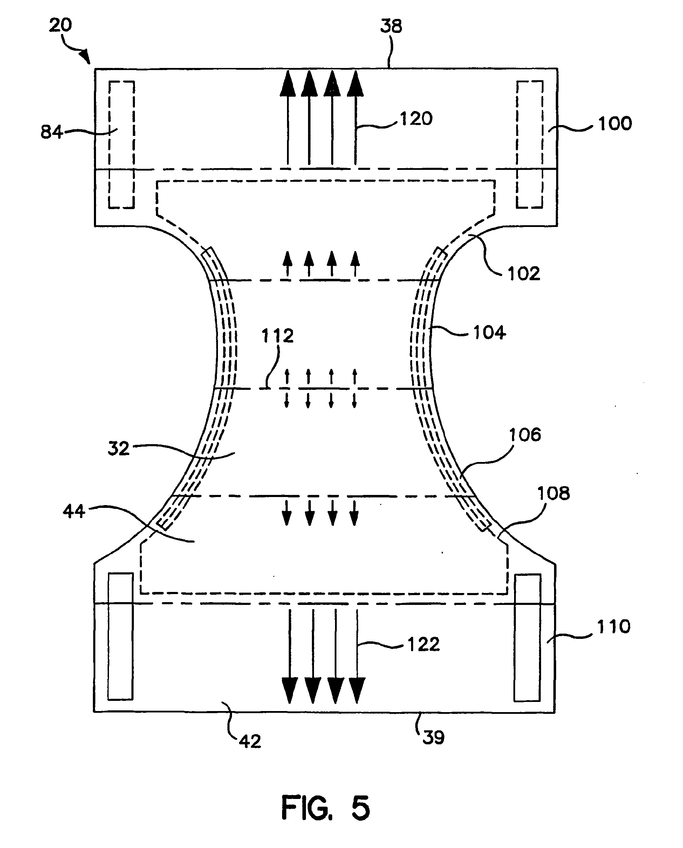

As described above, the present invention is particularly directed to absorbent articles having controlled stretch properties. For instance, referring to Fig. 5, the absorbent article 20 is shown in a flat state containing diagrammatical arrows illustrating the stretch properties of the chassis 32. As shown in Fig. 5, not only is the chassis 32 of the absorbent article 20 stretchable, but the amount of stretch in the article varies in the longitudinal direction. For instance, as illustrated, the chassis 32 of the absorbent article 20 for purposes of explanation is partitioned into six equal distant zones. The zones include a front waist zone 100, a front midsection zone 102, a front crotch zone 104, a back crotch zone 106, a back midsection zone 108, and a back waist zone 110. The front zones 100, 102 and 104 are separated from the back zones 106, 108 and 110 by a center line 112. In accordance with the present invention, the chassis 32 from the center line 112 to the front waist edge 38 has a total stretch of from about 5% to about 30%, such as from about 5% to about 25%. Similarly, the chassis 32, from the center line 112 to the back waist edge 39, may also have a total stretch of from about 5% to about 30%, such as from about 5% to about 25%. As also shown in Fig. 5 by the arrows 120 and 122, most of the stretch contained in the front zones or the back zones resides in the front waist zone 100 and in the back waist zone 110. In particular, at least 80%, such as greater than about 85%, or greater than about 90% of the total stretch contained in the front zones and the back zones may reside in the front waist zone 100 and the back waist zone 110, respectively. Thus, less than 20% of the total stretch in the front zones resides in the front midsection zone 102 and the front crotch zone 104, while less than 20% of the total stretch in the back zones resides in the back midsection zone 108 and the back crotch zone 106. For example, In one embodiment, the front waist zone 100 and/or the back waist zone 110 may have greater than about 4 times the stretch properties than the midsection zones and/or the crotch zones.

It should be understood that the above stretch properties of absorbent articles made in accordance with the present invention are contained in the chassis 32 in the longitudinal direction. As used herein, the stretch properties are Independent of the properties of any auxiliary components, such as flap elastics, elastic gasket components, or leg elastic components.

Through the above construction, the absorbent article 20 is provided with form-fitting properties that not only maximize comfort but also provide an aesthetically pleasing appearance when worn. The distribution of stretch properties, for instance, maintains the crotch region next to the wearer, even after the absorbent garment has been wetted. Further, the stretch properties facilitate donning of the article.

Various techniques may be used in order to produce the absorbent article 20 with the above stretch properties. In constructing absorbent articles In accordance with the present invention, for instance, the outer cover 40 is elastic, while the bodyside liner 42 Is stretchable. In other embodiments, both the outer cover and the bodyside liner may be elastic. Depending upon the construction of the article, the absorbent structure 44 may also be stretchable and/or elastic.

In one particular embodiment, the outer cover 40 is made from an elastic material and the bodyside liner **42** is made from stretchable and/or elastic materials. These materials are incorporated into the absorbent article **20** in a manner that provides the article with stretch characteristics in the longitudinal direction. In order to inhibit stretch in the midsection zones **102** and **108** and in the crotch zones **104** and **106,** an absorbant structure **44** is incorporated into the chassis **32** of the absorbent article **20** that has relatively low stretch properties. By attaching the absorbent structure **44** to the chassis **32** in the crotch zones and/or In the midsection zones, stretch in these zones becomes inhibited. Because the absorbent structure **44** is not attached to the waist zones **100** or **110,** however, the waist zones retain their stretch characteristics.

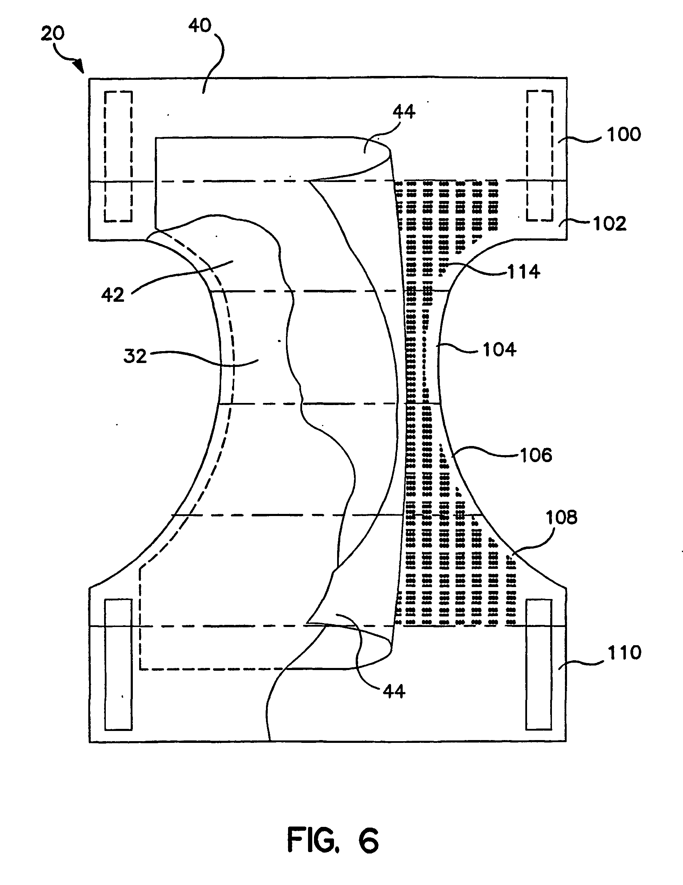

For example, referring to **Fig. 6,** an absorbent article **20** made in accordance with the present invention is shown. As illustrated, the chassis **32** of the absorbent article **20** is partitioned into six equal distant zones. The absorbent article **20** includes a stretchable outer cover **40** and a stretchable liner **42**. Positioned in between the outer cover **40** and the liner **42** is a substantially nonelastic absorbent structure **44**. The absorbent structure **44** is shown attached to the outer cover **40** using an adhesive **114.** By attaching the absorbent structure **44** to the outer cover **40,** the stretch properties of the outer cover **40** are inhibited in the midsection zones **102** and **108** and in the crotch zones **104** and **106.** Thus, an absorbent article **20** can be produced that not only has a total stretch of from about 5% to about 30% in the front zones and in the back zones, but also includes crotch zones and midsection zones that contain less than 20% of the total stretch of the article.

In accordance with the present invention, the adhesive **114** is applied in order to tailor the stretch properties to a particular application. For instance, as shown in **Fig. 6,** the adhesive **114** is applied in a pattern. In this embodiment, heavy amounts of adhesive are positioned in the crotch zones while lesser amounts of adhesive are applied in the midsection zones. In this manner, the crotch zones **104** and **106** have less stretch properties than the midsection zones **102** and **108.** Since no adhesive is applied to the waist zones **100** and **110,** however, most of the stretch characteristics reside in the waist zones. For instance, as shown in **Fig. 5,** when the chassis **32** of the absorbent article **20** is stretched in the longitudinal direction, the waist zones **100** and **110** stretch to a greater extent than the midsection zones **102** and **108.** Further, the midsection zones **102** and **108** may be engineered to have greater stretch characteristics than the crotch zones **104** and **106.** As one skilled in the art may appreciate, however, the adhesive **114** may be applied in other patterns for further altering the overall stretch properties of the product .

The adhesive **114** used to construct the absorbent article **20** may be any suitable adhesive for the application. For instance, in one embodiment, a hot melt adhesive may be used.

It should also be understood, that in addition to using the absorbent structure **44** to limit stretch within the chassis **32,** other components may be incorporated into the absorbent article for the purpose of limiting stretch. For instance, In other embodiments, a nonextensible material or a material with relatively low stretch characteristics may be incorporated into the absorbent article for the sole purpose of limiting stretch in the crotch zones and/or the midsection zones.

In another alternative arrangement, the outer cover **40,** the bodyside liner **42** and/or the absorbent structure **44** may be formed from multiple components and separate pieces that are attached together to form the absorbent article **20** having the desired stretch properties. In this embodiment, for instance, the outer cover **40** may be made from three separate pieces of material. Two pieces of material may be used, for instance, to construct the front waist zone **100** and the back waist zone **110.** These pieces of material may be stretchable and/or elastic. The midsection zones and the crotch zones of the outer cover **40,** however, may be made from a material that has lower stretch properties than the material used to form the waist zones. The three pieces of material may then be connected or attached together using any suitable attachment technique, such as thermal bonding or through the use of an adhesive. In this arrangement, the liner may also be made from three separate pieces of material that assist in matching the stretch characteristics of the outer cover **40.**

In other arrangements, the stretch properties of the chassis **32** may be primarily inhibited or restricted by the liner **42.**

In one embodiment, the stretch contained in the crotch zones **104** and **106** and/or in the midsection zones **102** and **108** only becomes active when the chassis **32** is placed under higher tensions, such as tensions higher than about 75 g/cm. At lower tensions, the crotch zones and the midsection zones may only be slightly stretchable or nonextensible. In this embodiment, the chassis **32** may be constructed by incorporating into one of the crotch zones or one of the midsection zones a material having a relatively high modulus that does not stretch until tensions reach a predetermined level.

In other arrangements, a low stretch or nonstretchable material is adhered to the outer cover **40** and/or the liner **42** to prevent stretch of the chassis **32** In the crotch zones and/or the midsection zones. The relatively low stretch material, however, may be attached to the chassis **32** In the midsection zones and/or in the crotch zones using an attachment structure that degrades or otherwise breaks down at higher tensions. For instance, the low stretch component may be adhered to the layers of the chassis using an adhesive, pressure bonding, or thermal bonding that secures the component to the layers at lower tensions but breaks free at higher tensions releasing greater stretch in the crotch zones and/or in the midsection zones.

As described above, the front zones of the chassis **32** and/or the back zones of the chassis **32** have a total stretch of from about 5% to about 30%. It should be understood that in certain embodiments it may be desirable to only have the stretch characteristics present in the front zones or only have the stretch characteristics present in the back zones. Further, it should also be understood that the stretch in the front zones may be greater or less than the stretch in the back zones. For instance, in one embodiment, the total stretch in the front zones may be less than about 20% while the total stretch in the back zones may be greater than about 20%. For instance, greater stretch may be needed in the back zones in order to provide a better fit around the buttocks of the wearer.

The outer cover **40**, the inner liner **42** and the absorbent structure **44** may be made from many different materials depending upon the particular application and the desired result. All three layers, for instance, may be stretchable and/or elastic. In accordance with the invention, the outer cover is elastic. Further, the stretch properties of each layer may vary In order to control the overall stretch properties of the product. For instance as described above, in some embodiments the absorbent structure may be used to limit stretch of the absorbent article in the midsection and crotch zones. In this embodiment, the absorbent structure may have little to no stretch properties or, at least, stretches a lesser amount than at least one of the other layers.

The outer cover **40** may be made from various materials. The outer cover **40**, for instance, may be breathable and/or may be liquid impermeable. The outer cover **40** may be constructed of a single layer, multiple layers, laminates, spunbond fabrics, films, meltblown fabrics, elastic netting, microporous webs, bonded card webs or foams provided by elastomeric or polymeric materials. The outer cover **40**, for instance, can be a single layer of a liquid impermeable material, or alternatively can be a multi-layered laminate structure in which at least one of the layers is liquid impermeable. In other embodiments, however, it should be understood that the outer cover may be liquid permeable. In this embodiment, for instance, the absorbent article may contain an interior liquid barrier layer.

For instance, the outer cover **40** can include a liquid permeable outer layer and a liquid impermeable inner layer that are suitably joined together by a laminate adhesive, ultrasonic bonds, thermal bonds, or the like. Suitable laminate adhesives, which can be applied continuously or intermittently as beads, a spray, parallel swirls, or the like, can be obtained from Bostik Findley Adhesives, Inc., of Wauwatosa, Wisconsin, U.S.A., or from National Starch and Chemical Company, Bridgewater, New Jersey U.S.A. The liquid permeable outer layer can be any suitable material and is desirably one that provides a generally cloth-like texture. One example of such a material is a 20 gsm (grams per square meter) spunbond polypropylene nonwoven web. The outer layer may also be made of those materials of which the liquid permeable bodyside liner **42** is made.

The inner layer of the outer cover **40** can be both liquid and vapor impermeable, or it may be liquid impermeable and vapor permeable. The Inner layer can be manufactured from a thin plastic film, although other flexible liquid impermeable materials may also be used. The inner layer, or the liquid impermeable outer cover **40** when a single layer, prevents waste material from wetting articles, such as bed sheets and clothing, as well as the wearer and caregiver. A suitable liquid impermeable film for use as a liquid impermeable inner layer, or a single layer liquid impermeable outer cover **40,** is a 0.02 millimeter polyethylene film commercially available from Pliant Corporation of Schaumburg, Illinois, U.S.A.

In embodiments of the invention, the outer cover **40** is stretchable and elastic. Elastic non-woven laminate webs that can be used as the outer cover **40** include a non-woven material joined to one or more gatherable non-woven webs, films, or foams. Stretch Bonded Laminates (SBL) and Neck Bonded Laminates (NBL) are examples of elastomeric composites. Non-woven fabrics are any web of material which has been formed without the use of textile weaving processes which produce a structure of individual fibers that are interconnected in an integrating manner.

Examples of suitable materials are spunbond-meltblown fabrics, spunbond-meltblown-spunbond fabrics, spunbond fabrics, or laminates of such fabrics with films, foams, or other nonwoven webs. Elastomeric materials may include cast or blown films, foams, meltblown fabrics or spunbond fabrics composed of polyethylene, polypropylene, or polyolefin elastomers, as well as combinations thereof. The elastomeric materials may include PEBAX elastomer (available from AtoChem located in Philadelphia, Pa.), HYTREL elastomeric polyester (available from Invista of Wilmington, Del.), KRATON elastomer (available from Kraton Polymers of Houston, Tex.), or strands of LYCRA elastomer (available from Invista of Wilmington, Del.), or the like, as well as combinations thereof. The outer cover 40 may include materials that have elastomeric properties through a mechanical process, printing process, heating process, or chemical treatment. For examples such materials may be apertured, creped, neck-stretched, heat activated, embossed, and micro-strained; and may be in the form of films, webs, and laminates.

In particular aspects of the invention, the outer cover **40** may include a 0.4 ounces per square yard (osy) (13.6 grams per square meter (gsm)) basis weight layer of G2760 KRATON elastomer strands adhesively laminated with a 0.3 gsm layer of adhesive between two facings. Each facing can be composed of a thermal point bonded bicomponent spunbond non-woven fibrous web having a 0.7 osy (23.7 gsm) basis weight. The adhesive is similar to an adhesive which is supplied by Bostik Findley Adhesive and designated as H2525 A, and the elastomer strands are placed and distributed to provide approximately 12 strands of KRATON elastomer per inch (2.54 cm) of lateral width of the outer cover **40.**

Alternatively, the outer cover **40** may include a woven or non-woven fibrous web layer which has been totally or partially constructed or treated to impart the desired levels of liquid Impermeability to selected regions that are adjacent or proximate the absorbent structure. For example, the outer cover **40** may include a gas-permeable, non-woven fabric layer laminated to a polymer film layer which may or may not be gas-permeable. Other examples of fibrous, cloth-like outer cover **40** materials can include a stretch thinned or stretch thermal laminate material composed of a 0.8 mil (0.015 mm) thick polypropylene blown film and a 0.7 osy (23.8 gsm) polypropylene spunbond material (2 denier fibers).

Suitable materials for a biaxially stretchable outer cover **40** include biaxially stretchable material and biaxially elastic stretchable material. One example of a suitable outer cover material can include a 0.3 osy (10.2 gsm) polypropylene spunbond that is necked 60% in the lateral direction **49** and creped 60% in the longitudinal direction **48,** laminated, with 3 grams per square meter (gsm) Findley 2525A styrene-isoprene-styrene based adhesive to 8 gsm PEBAX 2533 film with 20% $TiO_2$ concentrate. The outer cover **40** can suitably be stretched, laterally and/or longitudinally, by at least 30% (to at least 130% of an initial (unstretched) width and/or length of the outer cover **40**). More suitably, the outer cover 40 can be stretched laterally and/or longitudinally, by at least 50% (to at least 150% of the unstretched width or length of the outer cover **40**). Even more suitably, the outer cover **40** can be stretched, laterally and/or longitudinally, by at least 100% (to at least 200% of the unstretched width or length of the outer cover **40**). Tension force in the outer cover **40** at 50% extension is suitably between 50 and 1000 grams, more suitably between 100 and 600 grams, as measured on a 3 inch (7.62 cm) wide piece of the outer cover material.

Another example of a suitable material for a biaxially stretchable outer cover **40** is a breathable elastic film/nonwoven laminate, described in U.S. Patent No. 5,883,028, issued to Morman et al. Examples of materials having two-way stretchability and retractability are disclosed in U.S. Patent No. 5.116,662 issued to Morman and U.S. Patent No. 5,114,781 issued to Morman. These two patents describe composite elastic materials capable of stretching in at least two directions. The materials have at least one elastic sheet and at least one necked material, or reversibly necked material, joined to the elastic sheet at least at three locations arranged in a nonlinear configuration, so that the necked, or reversibly necked, web is gathered between at least two of those locations.

The bodyside liner **42** is suitably compliant, soft-feeling, and non-irritating to the wearer's skin. The bodyside liner **42** is also sufficiently liquid permeable to permit liquid body exudates to readily penetrate through its thickness to the absorbent structure **44.** A suitable bodyside liner **42** may be manufactured from a wide selection of web materials, such as porous foams, reticulated foams, apertured plastic films, woven and non-woven webs, or a combination of any such materials. For example, the bodyside liner **42** may include a meltblown web, a spunbonded web, or a bonded-carded-web composed of natural fibers, synthetic fibers or combinations thereof. The bodyside liner **42** may be composed of a substantially hydrophobic material, and the hydrophobic material may optionally be treated with a surfactant or otherwise processed to impart a desired level of wettability and hydrophilicity.

The bodyside liner **42** is stretchable, and more suitably it may be elastomeric. Suitable elastomeric materials for construction of the bodyside liner **42** can include elastic strands, LYCRA elastics, cast or blown elastic films, nonwoven elastic webs, meltblown or spunbond elastomeric fibrous webs, as well as combinations thereof. Examples of suitable elastomeric materials include KRATON elastomers, HYTREL elastomers, ESTANE elastomeric polyurethanes (available from Noveon of Cleveland, Ohio), or PEBAX elastomers.

As an additional example, in one aspect the bodyside liner **42** suitably includes a non-woven, spunbond polypropylene fabric composed of about 2 to 3 denier fibers formed into a web having a basis weight of about 12 gsm which is necked approximately 60 percent. Strands of about 9 gsm KRATON G2760 elastomer material placed eight strands per inch (2.54 cm) are adhered to the necked spunbond material. The fabric Is surface treated with an operative amount of surfactant, such as about 0.6 percent AHCOVEL Base N62 surfactant, available from ICI Americas, a business having offices in Wilmington, Del., U.S.A. The surfactant can be applied by any conventional means, such as spraying, printing, brush coating or the like. Other suitable materials may be extensible biaxially stretchable materials, such as a neck stretched/creped spunbond. The bodyside liner **42** can also be made from extensible materials as are described in U.S. Patent No. 6,552,245 (Application Serial No. 09/563,417) filed on May 3, 2000 by Roessier et al. The bodyside liner 42 can also be made from biaxially stretchable materials as are described in U.S. Patent No. 6,642,134 (Application Serial No. 09/698,512) filed on October 27, 2000 by Vukos et al.

The liner **42** can suitably be stretched, laterally and/or longitudinally, by at least 30% (to at least 130% of an initial (unstretched) width and/or length of the liner **42).** More suitably, the liner **42** can be stretched laterally and/or longitudinally, by at least 50% (to at least 150% of the unstretched width or length of the liner **42**). Even more suitably, the liner **42** can be stretched, laterally and/or longitudinally, by at least 100% (to at least 200% of the unstretched width or length of the liner **42**). Tension force in the liner **42** at 50% extension is suitably between 50 and 1000 grams, more suitably between 100 and 600 grams, as measured on a 3 inch (7.62 cm) wide piece of the liner material.

The absorbent structure **44** is disposed between the outer cover 40 and the bodyside liner **42**. The absorbent structure **44** can be any structure or combination of components which are generally compressible, conformable, non-irritating to a wearer's skin, and capable of absorbing and retaining liquids and certain body wastes. For example, the absorbent structure **44** may include an absorbent web material of cellulosic fibers (e.g., wood pulp fibers), other natural fibers, synthetic fibers, woven or nonwoven sheets, scrim netting or other stabilizing structures, superabsorbent material, binder materials, surfactants, selected hydrophobic materials, pigments, lotions, odor control agents or the like, as well as combinations thereof. In a particular aspect, the absorbent web material is a matrix of cellulosic fluff and superabsorbent hydrogel-forrming particles. The cellulosic fluff may include a blend of wood pulp fluff. One preferred type of fluff is identified with the trade designation CR 1654, available from Bowater of Greenville, South Carolina, USA, and is a bleached, highly absorbent sulfate wood pulp containing primarily southern soft wood fibers. The absorbent materials may be formed into a web structure by employing various conventional methods and techniques. For example, the absorbent web may be formed with a dry-forming technique, an air forming technique, a wet-forming technique, a foam-forming technique, or the like, as well as combinations thereof. Methods and apparatus for carrying out such techniques are well known in the art. Furthermore, the absorbent structure may itself encompass multiple layers in the Z direction. Such multiple layers may take advantage of differences in absorbency capacity, such as by placing a lower capacity absorbent material layer closer to the liner **42** and a higher capacity absorbent material closer to the outer cover layer **40.** Likewise, discrete portions of an absorbent single-layered structure may encompass higher capacity absorbents, and other discrete portions of the structure may encompass lower capacity absorbents.

As a general rule, the superabsorbent material is present in the absorbent web in an amount of from about 0 to about 90 weight percent based on total weight of the web. The web may have a density within the range of about 0.10 to about 0.60 grams per cubic centimeter.

Superabsorbent materials are well known in the art and can be selected from natural, synthetic, and modified natural polymers and materials. The superabsorbent materials can be inorganic materials, such as silica gels, or organic compounds, such as crosslinked polymers. Typically, a superabsorbent material is capable of absorbing at least about 10 times its weight in liquid, and desirably is capable of absorbing more than about 25 times its weight in liquid. Suitable superabsorbent materials are readily available from various suppliers. For example, SXM 9394, and Favor 9543 superabsorbents are available from DeGussa Superabsorbers.

After being formed or cut into a desired shape, the absorbent web material may be wrapped or encompassed by a suitable tissue or meltblown web or the like wrap sheet that aids in maintaining the integrity and shape of the absorbent structure **44.**

The absorbent web material may also be a coform material. The term "coform material" generally refers to composite materials comprising a mixture or stabilized matrix of thermoplastic fibers and a second non-thermoplastic material. As an example, coform materials may be made by a process in which at least one meltblown die head is arranged near a chute through which other materials are added to the web while it is forming. Such other materials may include, but are not limited to, fibrous organic materials such as woody or non-woody pulp such as cotton, rayon, recycled paper, pulp fluff and also superabsorbent particles, inorganic absorbent materials, treated polymeric staple fibers and the like. Any of a variety of synthetic polymers may be utilized as the melt-spun component of the coform material. For instance, in certain aspects, thermoplastic polymers can be utilized. Some examples of suitable thermoplastics that can be utilized Include polyolefins, such as polyethylene, polypropylene, polybutylene and the like; polyamides; and polyesters. In one aspect, the thermoplastic polymer is polypropylene. Some examples of such coform materials are disclosed in U.S. Patent Nos. 4,100,324 to Anderson, et al.; 5,284,703 to Everhart, et al.; and 5,350,624 to Georger, et al.

In a particular aspect of the absorbent articles of the present invention, the absorbent structure **44** may also be elastomeric. For this purpose, the absorbent web material can include elastomeric fibers in an amount which is at least a minimum of about 2 wt %. The amount of elastomeric fibers can alternatively be at least about 3 wt %, and can optionally be at least about 5 wt % to provide improved performance. In addition, the amount of elastomeric fibers can be not more than about 60 wt %. Alternatively, the amount of elastomeric fibers can be not more than about 45 wt %, and optionally, can be not more than about 30 wt % to provide improved benefits. These values may impact the absorbent structure **44** by affecting the desired levels of stretchability and structural stability without excessively degrading the physical properties or the liquid-management properties of the absorbent structure. An absorbent web material with an excessively low proportion of elastomeric fibers may be insufficiently stretchable, and a web material with an excessively high proportion of elastomeric fibers may exhibit an excessive degradation of its absorbency functionalities, such as poor Intake, poor distribution, poor retention of liquid.

The absorbent structure **44** may include an elastomeric coform absorbent web material. Such materials are described for instance in US 6,231,557 B1 and 6,362,369 B1. In particular aspects, the elastomeric coform material can have an overall coform basis weight of at least about 50 gsm, such as up to about 1200 gsm. The coform basis weight, for example, may be at least about 100 gsm, such as at least about 200 gsm. These values can provide the absorbent structure with the desired stretchability and structural stability without excessively degrading the physical properties or the liquid-management functionalities of the absorbent structure.

For example, retention portions having excessively low proportions of elastomeric coform material may not be sufficiently stretchable. Conversely, an absorbent web material having excessively large amounts of elastomeric coform materials can exhibit an excessive degradation of their absorbency functionalities, such as, an excessive degradation of Intake, distribution and/or retention properties.

Other examples of usable elastomeric absorbent bodies are described in international patent application WO 03/051254 and U.S. Patent Nos. 5,964,743, 5,645,542, 6,231,557, and 6,362,389 B1.

**Testing Procedures**

For the purposes of the present invention, the properties, such as tensile strength, tensile load and elongation, of a material or component can be determined by ASTM Procedure D 3039 "Tensile Properties of polymer Matrix Composite Materials", with the following specification and particulars:

Test facility environment: 23 +-.1 degree C., and relative humidity of 50 +-.2 percent;
Gauge length: 50.8 mm;
Specimen width (aligned perpendicular to the applied force): 3 inches (76.2 mm); Jaw width (measured parallel to the applied force): 1/2 inch (12.7 mm); Jaw length (measured perpendicular to the applied force): 4 inches (101.8 mm); Jaw speed: 500 mm/min.

A suitable testing device is a SINTECH constant rate of extension tensile tester (available from MTS Systems Corporation, a business having offices located in Edens Prairie, Minnesota), or an equivalent device. The tensile tester is operatively programmed with suitable software, such as TESTWORKS software (available from MTS Systems Corporation), or an equivalent software.

Test specimens may include a single component, multi-components or multi-layered structure and are stapled at their length-wise ends at locations outside of the test area to prevent slippage of the individual materials within the composite when the composite is mounted in the jaws of the testing device.

The 3-inch (76.2 mm) width of each specimen can be cut with a JDC precision cutter (available from Thwing-Albert Instrument Company, a business having offices located in Philadelphia, Pa.), or an equivalent device.

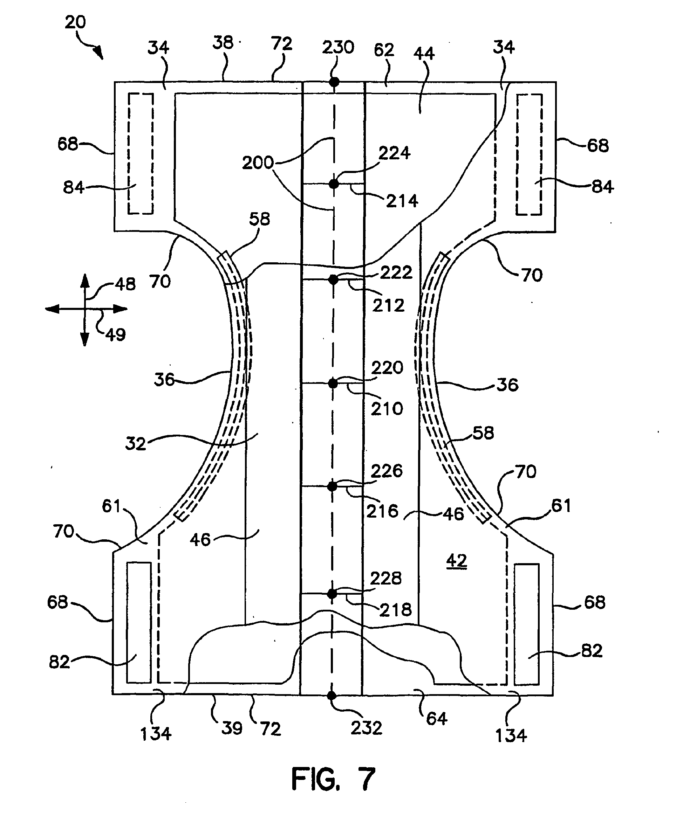

Test specimens are removed from an individual absorbent article **20,** each test specimen is desirably removed from the absorbent article **20** at a location which is centered along the longitudinal centerline **200,** as representatively shown in **Fig. 7.** The specimen locations are selected to avoid the effects of auxiliary elasticized components that may be present; such as elasticized leg bands, elasticized waistbands, elasticized containment flaps and the like. In certain applications, any of the above described auxiliary elasticized components may be removed from the absorbent article prior to obtaining samples for testing or, alternatively, may be inactivated by cutting the auxiliary elastic components every one centimeter along the length of the component.

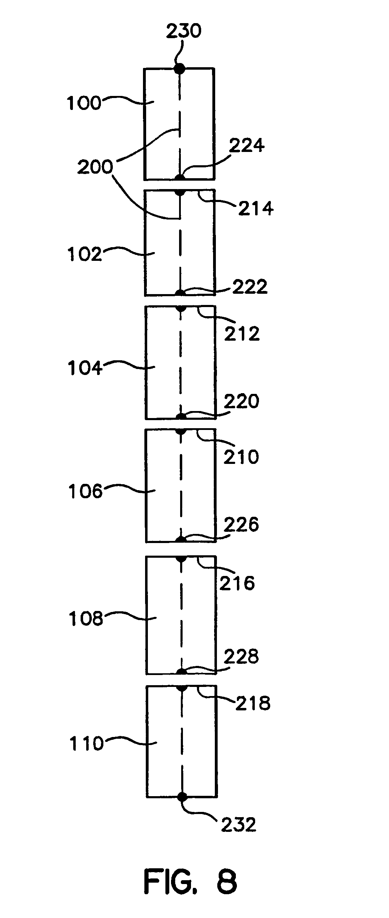

As shown in **Fig. 8,** once a strip is obtained from an individual absorbent article, the strip is severed into six equidistant sections. The sections include a front waist zone **100,** a front midsection zone **102,** a front crotch zone **104,** a back crotch zone **106,** a back midsection zone **108,** and a back waist zone **110.** Each of the individual sections or zones are then tested using the above described testing device.

Three specimens are tested per sample section. More than one absorbent article may be needed in order to obtain the specimens. The reported value of the particular property being determined for each sample is the arithmetic average of the corresponding data points measured for the three specimens during the testing.

The following data point is recorded for each test specimen:

Elongation at 100 g load per centimeter width (%);

The percent elongation can be determined in accordance with the following formula:

$$\% \text{ elongation} = (L_f - L_0) * 100 / L_0 \; ;$$

where:

$L_0$ = initial, non-elongated length
$L_f$ = final, elongated length at 100 gram load per centimeter width.

Once the stretch characteristics are known for each of the six sections **100, 102, 104, 106, 108,** and **110,** total stretch of the front zones and/or the back zones may be calculated. The percent stretch in each section may also be determined. As described above, absorbent articles made according to the present invention have greater stretch characteristics in the front waist zone **100** and/or the back waist zone **110** in comparison to the other zones.

Specimen Preparation

1. The absorbent article **20** is clamped in a conventional vertical lightbox by hanging the article vertically with the back waist edge **39** of the article is at the top of the lightbox and the liner **42** of the article facing the viewer.
2. The top clamp is laterally centered on the end edge of the article.

3. A bottom clamp weighing 200 grams is attached to the front waist edge 38 of the article and allowed to hang freely.

4. With the article hanging, the following measurements and markings as illustrated in **Fig. 7** are determined and marked on the liner **42** of the article.

5. Construct the longitudinal centerline **200** of the article by measuring and marking the total width of the article in the lateral direction between outer edges **68** in the front and back regions **22** and **24,** marking centerpoints In both regions **22** and **24.**

6. Construct the central lateral centerline **210** of the article by measuring the total length of the longitudinal centerline **200,** marking the centerpoint **220** at the point that equals the total length of the longitudinal centerline **200** divided by two.

7. Measure the distance from centerpoint **220** to the end of the longitudinal length at the front waist edge **38,** at centerpoint **230.** Mathematically divide this distance by three and mark two centerpoints **222** and **224** equidistant from each other such that three equal length sample sections are generated. Construct the front midsection lateral centerline **214** and the front waist lateral centerline **212.**

8. Measure the distance from centerpoint **220** to the end of the longitudinal length at the back waist edge **39** at centerpoint **232.** Mathematically divide this distance by three and mark two centerpoints **226** and **228** equidistant from each other such that three equal length sample sections are generated. Construct the-back midsection lateral centerline **218** and the back waist lateral centerline **216.**

9. Mark each of the six test specimens based on location and cut them away from the absorbent article as shown in **Fig. 8.**

10. If the six test specimens have a length that is less than 50.8 mm, the gauge length may be reduced in order to test the specimens. The gauge length should not be less than 25.4 mm.

11. If the width of the central portion of the article is less than 76.2 mm after avoiding the auxiliary components by either removing them or rendering them inactive, narrower sections can be cut and the procedure and analysis be adjusted to the narrower width. The central portion width should be no narrower than 25.4 mm.

These and other modifications and variations to the present invention may be practiced by those of ordinary skill in the art, without departing from the scope of the present invention, which is more particularly set forth in the appended claims. In addition, it should be understood that aspects of the various embodiments may be interchanged both in whole or in part. Furthermore, those of ordinary skill in the art will appreciate that the foregoing description is by way of example only, and is not intended to limit the invention so further described in such appended claims.

## Claims

1. An absorbent article (20) comprising:

a stretchable outer cover (40) comprising an elastic material;
a stretchable bodyside liner (42) joined to the outer cover in a superimposed relation;
an absorbent structure (44) positioned in between the outer cover and the liner; and
wherein the outer cover (40), liner (42) and absorbent structure (44) form a chassis (32), the chassis defining a waist opening (50) opposite two leg openings (52) when worn about a wearer;

**characterized in that** the stretch In the crotch zone and the midsection zone is reduced by attaching the absorbent structure (44) to the liner (42), the outer cover (40) or to both the liner (42) and the outer cover (40), and wherein the absorbent structure (44) is attached to the liner (42), the outer cover (40) or to both the liner and the outer cover using an adhesive;

the chassis (32) comprising six equal distant zones in a longitudinal direction, the zones including a front waist zone (100), a back waist zone (110), a front midsection zone (102), a back midsection zone (108), a front crotch zone (104), and a back crotch zone (106), arid wherein the adhesive is applied in varying amounts in order to control stretch properties of the chassis in the longitudinal direction, wherein the chassis is constructed such that at least one of the front zones or the back zones have a lengthwise stretch of from 5% to 30% as measured according to the test procedure described in the section entitled "Testing Procedures" above, and wherein at least 60% of the above stretch is contained in the respective waist zone.

2. An absorbent article as defined in claim 1, wherein the chassis (32) is constructed such that the back crotch zone (106), the back midsection zone (108) and the back waist zone (110) have the lengthwise stretch of from 5% to 30% as measured according to the test procedure described in the section entitled testing Procedures" above, and wherein at least 80% of the above stretch is contained in the back waist zone.

3. An absorbent article as defined in claim 1, wherein the chassis (32) is constructed such that the front crotch zone (104), the front midsection zone (102) and the front waist zone (100) have the lengthwise stretch of from 5% to 30% as measured according to the test procedure described in the section entitled "Testing Procedures" above, and wherein at least 80% of the above stretch is contained in the front waist zone.

4. An absorbent article as defined in any of the preceding claims, wherein at least 80% of the stretch is contained in the respective waist zone.

5. An absorbent article as defined in any of the preceding claims, wherein at least 90% of the stretch is contained in the respective waist zone.

6. An absorbent article as defined in claim 1, wherein both the front zones and the back zones have a lengthwise stretch of from 5% to 30% as measured according to the test procedure described in the section entitled "Testing Procedures" above, and wherein at least 60% of the stretch in the front zones is contained In the front waist zone and wherein at least 60% of the stretch in the back zones is contained in the back waist zone.

7. An absorbent article as defined in any of the preceding claims, wherein the back zones have greater stretch than the front zones.

8. An absorbent article as defined in claim 7, wherein the back zones have at least 5% more stretch than the front zones.

9. An absorbent article as defined in any of the preceding claims, wherein the absorbent structure (44) is located in the front and back crotch zones (108, 104) and at least partially in the front midsection zone (102).

10. An absorbent article as defined in any of claims 3 or 6, wherein the front crotch zone (104) is only stretchable when placed under a longitudinal, tension of greater than 75 g/cm.

11. An absorbent article as defined in any of the preceding claims, wherein the liner (42) is elastic,

12. An absorbent article as defined in any of the preceding claims, wherein the outer cover (40) is constructed from multiple pieces of material In the longitudinal direction.

13. An absorbent article as defined in any of the preceding claims, wherein the outer cover (40) comprises an elastic laminate containing a nonwoven web.

14. An absorbent article as defined in any of the preceding claims, wherein the liner (42) comprises a liquid permeable elastic film or a nonwoven web containing an elastic material.

15. An absorbent article as defined in any of the preceding claims, wherein the absorbent structure (44) is substantially non-elastic.

16. An absorbent article as defined in any of claims 1 through 9. wherein the front and back crotch zones have substantially no stretch when placed under lower longitudinal tensions, at tensions greater than 75 g/cm, however, an attachment of the absorbent structure to the chassis at least partially breaks down releasing stretch within the front and back crotch zones.

**Patentansprüche**

1. Ein absorbierender Artikel (20) umfassend:

eine dehnbare äußere Deckschicht (40), die ein elastisches Material umfasst;
eine dehnbare körperseitige Auskleidung (42), die mit der äußeren Deckschicht in einer darüberliegenden Beziehung verbunden ist;
eine absorbierende Struktur (44), die zwischen der äußeren Deckschicht und der Auskleidung angeordnet ist; und
wobei die äußere Deckschicht (40), die Auskleidung (42) und die absorbierende Struktur (44) einen Rahmen (32) bilden, wobei der Rahmen eine Hüftöffnung (50) gegenüberliegend zu zwei Beinöffnungen (52) definiert, wenn durch einen Träger getragen;
**dadurch gekennzeichnet, dass** die Dehnbarkeit in dem Schrittbereich und dem Mittenabschnittsbereich reduziert ist durch Anbringen der absorbierenden Struktur (44) an der Auskleidung (42), der äußeren Deckschicht (40) oder sowohl der Auskleidung (42) als auch der äußeren Deckschicht (40) und wobei die absorbierende Struktur (44) an der Auskleidung (42), der äußeren Deckschicht (40) oder sowohl der Auskleidung als auch der äußeren Deckschicht unter Verwendung eines Haftmittels angebracht ist;
wobei der Rahmen (32) sechs gleich ferne Bereiche in einer longitudinalen Richtung umfasst, wobei die Bereiche einen vorderen Hüftbereich (100), einen hinteren Hüftbereich (110), einen vorderen Mittenabschnittsbereich (102), einen hinteren Mittenabschnittsbereich (108), einen vorderen Schrittbereich (104) und einen hinteren Schrittbereich (106) umfassen und wobei das Haftmittel in variierenden Mengen angewendet ist, um die Dehneigenschaften des Rahmens in Längsrichtung zu kontrollieren, wobei der Rahmen konstruiert ist, so dass mindestens einer von den vorderen Bereichen oder den hinteren Bereichen eine Längsdehnbarkeit von von 5% bis 30% aufweisen, gemessen nach dem Testverfahren, das in dem Abschnitt mit dem Titel "Testing Procedures" weiter oben beschrieben ist, und wobei mindestens 60% der obigen Dehnbarkeit in dem jeweiligen Hüftbereich enthalten ist.

2. Ein absorbierender Artikel nach Anspruch 1, wobei der Rahmen (32) konstruiert ist, so dass der hintere Schrittbereich (106), der hintere Mittenabschnittsbereich (108) und der hintere Hüftbereich (110) die Längsdehnbarkeit von von 5% bis 30% aufweisen, gemessen nach dem Testverfahren, das in dem Abschnitt mit dem Titel "Testing Procedures" weiter oben beschrieben ist, und wobei mindestens 80% der obigen Dehnbarkeit in dem hinteren Hüftbereich enthalten ist.

3. Ein absorbierender Artikel nach Anspruch 1, wobei der Rahmen (32) konstruiert ist, so dass der vordere Schrittbereich (104), der vordere Mittenabschnittsbereich (102) und der vordere Hüftbereich (100) die Längsdehnbarkeit von von 5% bis 30% aufweisen, gemessen nach dem Testverfahren, das in dem Abschnitt mit dem Titel "Testing Procedures" weiter oben beschrieben ist, und wobei mindestens 80% der obigen Dehnbarkeit in dem vorderen Hüftbereich enthalten ist.

4. Ein absorbierender Artikel nach einem der vorhergehenden Ansprüche, wobei mindestens 80% der Dehnbarkeit in dem jeweiligen Hüftbereich enthalten ist.

5. Ein absorbierender Artikel nach einem der vorhergehenden Ansprüche, wobei mindestens 90% der Dehnbarkeit in dem jeweiligen Hüftbereich enthalten ist.

6. Ein absorbierender Artikel nach Anspruch 1, wobei sowohl die vorderen als auch die hinteren Bereiche eine Längsdehnbarkeit von von 5% bis 30% aufweisen, gemessen nach dem Testverfahren, das in dem Abschnitt mit dem Titel "Testing Procedures" weiter oben beschrieben ist, und wobei mindestens 60% der Dehnbarkeit in den vorderen Bereichen in dem vorderen Hüftbereich enthalten ist und wobei mindestens 60% der Dehnbarkeit in den hinteren Bereichen in dem hinteren Hüftbereich enthalten ist.

7. Ein absorbierender Artikel nach einem der vorhergehenden Ansprüche, wobei die hinteren Bereiche eine größere Dehnbarkeit aufweisen als die vorderen Bereiche.

8. Ein absorbierender Artikel nach Anspruch 7, wobei die hinteren Bereiche mindestens 5% mehr Dehnbarkeit aufweisen als die vorderen Bereiche.

9. Ein absorbierender Artikel nach einem der vorhergehenden Ansprüche, wobei die absorbierende Struktur (44) in dem vorderen und dem hinteren Schrittbereich (108, 104) und zumindest teilweise in dem vorderen Mittenabschnittsbereich (102) angeordnet ist

10. Ein absorbierender Artikel nach einem der Ansprüche 3 oder 6, wobei der vordere Schrittbereich (104) nur dehnbar ist, wenn unter einer Längsspannung von mehr als 75 g/cm gesetzt.

11. Ein absorbierender Artikel nach einem der vorhergehenden Ansprüche, wobei die Auskleidung (42) elastisch ist.

12. Ein absorbierender Artikel nach einem der vorhergehenden Ansprüche, wobei die äußere Deckschicht (40) aus vielen Materialteilen in der Längsrichtung konstruiert ist.

13. Ein absorbierender Artikel nach einem der vorhergehenden Ansprüche, wobei die äußere Deckschicht (40) ein elastisches, Vlies enthaltendes Laminat umfasst.

14. Ein absorbierender Artikel nach einem der vorhergehenden Ansprüche, wobei die Auskleidung (42) einen flüssigkeitsdurchlässigen elastischen Film oder ein Vlies umfasst, das elastisches Material enthält.

15. Ein absorbierender Artikel nach einem der vorhergehenden Ansprüche, wobei die absorbierende Struktur (44) im Wesentlichen nicht-elastisch ist.

16. Eine absorbierende Artikel nach einem der Ansprüche 1 bis 9, wobei der vordere und hintere Schrittbereich im Wesentlichen keine Dehnbarkeit aufweisen, wenn unter niedrigere Längsspannungen gesetzt, bei Spannungen größer als 75 g/cm jedoch eine Anbringung der absorbierenden Struktur an den Rahmen zumindest teilweise zusammenbricht und Dehnbarkeit innerhalb des vorderen und hinteren Schrittbereichs freigibt.

**Revendications**

1. Article absorbant (20) comprenant :

une feuille extérieure de couverture (40), extensible, comprenant un matériau élastique ;
une doublure côté corporel (42), extensible, réunie à la feuille extérieure de couverture selon une relation superposée ;
une structure absorbante (44) positionnée entre la feuille extérieure de couverture et la doublure ;

article dans lequel la feuille extérieure de couverture (40), la doublure (42) et la structure absorbante (44) forment une construction (32), la construction définissant une ouverture de ceinture (50) à l'opposé de deux ouvertures de jambe (52) lorsque l'article est en place sur un porteur ;

**caractérisé en ce que** l'extensibilité dans la zone d'entrejambe et la zone médiane est réduite en fixant la structure absorbante (44) sur la doublure (42) ou la feuille extérieure de couverture (40), ou à la fois sur la doublure (42) et la feuille extérieure de couverture (40), la structure absorbante (44) étant fixée à la doublure (42) ou à la feuille extérieure de couverture (40), ou à la fois à la doublure et à la feuille extérieure de couverture en utilisant un adhésif ; la construction (32) comprenant six zones équidistantes dans une direction longitudinale, les zones comprenant une zone de ceinture avant (100), une zone de ceinture arrière (110), une zone médiane avant (102), une zone médiane arrière (108), une zone avant d'entrejambe (104) et une zone arrière d'entrejambe (106), l'adhésif étant appliqué en quantité variable pour commander les propriétés d'extensibilité de la construction dans la direction longitudinale, la construction étant construite de telle sorte que l'une au moins des zones avant et des zones arrière a une extensibilité, dans la direction longitudinale, comprise entre 5 % et 30 % telle que mesurée selon la procédure d'essai décrite dans la section intitulée "Procédures d'essai" ci-avant, au moins 60 % de l'extensibilité mentionnée ci-dessus résidant dans la zone de ceinture respective.

2. Article absorbant selon la revendication 1, dans lequel la construction (32) est construite de telle sorte que la zone arrière d'entrejambe (106), la zone médiane arrière (108) et la zone de ceinture arrière (110) ont l'extensibilité, dans la direction longitudinale, comprise entre 5 % et 30 % telle que mesurée selon la procédure d'essai décrite dans la section intitulée "Procédures d'essai" ci-avant, au moins 80 % de l'extensibilité mentionnée ci-dessus résidant dans la zone de ceinture arrière.

3. Article absorbant selon la revendication 1, dans lequel la construction (32) est construite de telle sorte que la zone avant d'entrejambe (104), la zone médiane avant (102) et la zone de ceinture avant (100) ont l'extensibilité, dans la direction longitudinale, comprise entre 5 % et 30 % telle que mesurée selon la procédure d'essai décrite dans la section intitulée "Procédures d'essai" ci-avant, au moins 80 % de l'extensibilité mentionnée ci-dessus résidant dans la zone de ceinture avant.

4. Article absorbant selon l'une quelconque des revendications précédentes, dans lequel au moins 80 % de l'extensibilité résident dans la zone de ceinture respective.

5. Article absorbant selon l'une quelconque des revendications précédentes, dans lequel au moins 90 % de l'extensibilité résident dans la zone de ceinture respective.

6. Article absorbant selon la revendication 1, dans lequel tant les zones avant que les zones arrière ont une extensibilité, dans la direction longitudinale, comprise entre 5 % et 30 % telle que mesurée selon la procédure d'essai décrite dans la section intitulée "Procédures d'essai" ci-avant, et dans lequel au moins 60 % de l'extensibilité dans les zones avant résident dans la zone de ceinture avant, et au moins 60 % de l'extensibilité dans les zones arrière résident dans la zone de ceinture arrière.

7. Article absorbant selon l'une quelconque des revendications précédentes, dans lequel l'extensibilité des zones arrière est supérieure à celle des zones avant.

8. Article absorbant selon la revendication 7, dans lequel l'extensibilité des zones arrière est au moins 5 % supérieure à celle des zones avant.

9. Article absorbant selon l'une quelconque des revendications précédentes, dans lequel la structure absorbante (44) est située dans les zones avant et arrière d'entrejambe (106,104) et au moins partiellement dans la zone médiane avant (102).

10. Article absorbant selon la revendication 3 ou 6, dans lequel la zone avant d'entrejambe (104) n'est extensible que lorsqu'elle est placée sous une tension longitudinale supérieure à 75 g/cm.

11. Article absorbant selon l'une quelconque des revendications précédentes, dans lequel la doublure (42) est élastique.

12. Article absorbant selon l'une quelconque des revendications précédentes, dans lequel la feuille extérieure de couverture (40) est faite de pièces multiples de matériau dans la direction longitudinale.

13. Article absorbant selon l'une quelconque des revendications précédentes, dans lequel la feuille extérieure de couverture (40) comprend un stratifié élastique contenant un voile non-tissé.

14. Article absorbant selon l'une quelconque des revendications précédentes, dans lequel la doublure (42) comprend un film élastique perméable aux liquides ou un voile non-tissé contenant un matériau élastique.

15. Article absorbant selon l'une quelconque des revendications précédentes, dans lequel la structure absorbante (44) est sensiblement inélastique.

16. Article absorbant selon l'une quelconque des revendications 1 à 9, dans lequel les zones avant et arrière d'entrejambe sont sensiblement dépourvues d'extensibilité lorsqu'elles sont placées sous des tensions longitudinales inférieures, une fixation de la structure absorbante à la construction se rompant, cependant, au moins partiellement, à des tensions supérieures à 75 g/cm, en libérant l'extensibilité au sein des zones avant et arrière d'entrejambe.

FIG. 1

FIG. 2

FIG. 3

FIG. 4

**FIG. 5**

**FIG. 6**

FIG. 7

FIG. 8

**EP 1 746 959 B1**

**REFERENCES CITED IN THE DESCRIPTION**

**Patent documents cited in the description**

- WO 9722318 A
- WO 03057106 A
- US 5890627 A
- WO 0037009 A, A. Fletcher
- US 4940464 A, Van Gompel
- US 5766389 A, Brandon
- US 6645190 B, Olson
- US 4704116 A, Enloe
- US 5224405 A, Pohjola
- US 5104116 A, Pohjola
- US 5046272 A, Vogt
- US 4663220 A, Wisneski
- US 5226992 A, Morman
- EP 0217032 A, Taylor
- WO 0188245 A, Weich
- US 5486166 A
- US 5490846 A
- US 5820973 A

- US 5883028 A, Morman
- US 5116662 A, Morman
- US 5114781 A, Morman
- US 6552245 B
- US 09563417 B
- US 6642134 B
- US 09698512 B
- US 4100324 A, Anderson
- US 5284703 A, Everhart
- US 5350624 A, Georger
- US 6231557 B1
- US 6362369 B1
- WO 03051254 A
- US 5964743 A
- US 5645542 A
- US 6231557 B
- US 6362389 B1